# EUROPÄISCHE PATENTSCHRIFT

(11) **EP 1 272 462 B1**
(45) Veröffentlichungstag und Bekanntmachung des Hinweises auf die Patenterteilung: **11.01.2006**
(21) Anmeldenummer: 01927904.1
(22) Anmeldetag: 10.04.2001
(51) Int. Cl.: C07C 315/00, C07C 317/22

(54) **SEMIKONTINUIERLICHES VERFAHREN ZUR HERSTELLUNG VON 4,4'-DIHYDROXYDIPHENYLSULFON**
SEMI-CONTINUOUS METHOD FOR PRODUCING 4,4'-DIHYDROXYDIPHENYLSULFONE
PROCEDE SEMICONTINU POUR PRODUIRE 4,4'-DIHYDROXYDIPHENYLSULFONE

(30) Priorität: 14.04.2000 DE 10018580
(43) Veröffentlichungstag der Anmeldung: 08.01.2003
(73) Patentinhaber: BASF Aktiengesellschaft, 67056 Ludwigshafen (DE)
(72) Erfinder: PABST, Gunther, 68165 Mannheim (DE); KAST, Jürgen, 67459 Böhl-Iggelheim (DE)
(86) Internationale Anmeldenummer: PCT/EP2001/004081
(87) Internationale Veröffentlichungsnummer: WO 2001/079163

(56) Entgegenhaltungen:
- US-A- 3 297 766
- L.E. HINKEL, ET AL.: "4:4'- and 2:4'-dihydroxydiphenyl sulphones" JOURNAL OF THE CHEMICAL SOCIETY, 1949, Seiten 2854-2856, XP002172779 Royal Society of Chemistry, Letchworth, GB

## Beschreibung

Die vorliegenden Erfindung betrifft ein verbessertes semikontinuierliches Verfahren zur Herstellung von 4,4'-Dihydroxydiphenylsulfon mit den Schritten
(a) Umsetzen von Phenol mit einem Sulfonierungsmittel,
(b) Suspendieren des entstandenen Rohproduktes in mindestens 40°C heißem Wasser, welches frei von inerten organischen Lösungsmitteln ist und Restmengen an nicht umgesetztem Phenol enthalten kann, und Abfiltrieren des Produktes sowie
(c) Rückführen der entstehenden edukt- und/oder produkthaltigen Abfallströme in den Herstellprozeß.

4,4'-Dihydroxydiphenylsulfon ("4,4'-Bisphenol S") ist unter anderem als galvanotechnischer Hilfsstoff, als Rohmaterial für die Herstellung von Kondensationsharzen, beispielsweise zum Einsatz als Ledergerbstoffe, und für die Herstellung von Fasern und insbesondere Kunststoffen wie Polyethersulfonen von großem wirtschaftlichem Interesse. Da die Eigenschaften der aus 4,4'-Dihydroxydiphenylsulfon hergestellten Polymeren stark vom Reinheitsgrad und vom Isomerenverhältnis der eingesetzten Monomere abhängen, sind selektive Syntheseverfahren für 4,4'-Dihydroxydiphenylsulfon gesucht. Desweiteren sind aus wirtschaftlichen und ökologischen Gründen Syntheseverfahren mit geringsten oder keinen Abfallströmen erwünscht.

Syntheseverfahren, die brennbare inerte organische Lösungsmittel wie Methanol, Ethanol, o-Dichlorbenzol oder Toluol bei der Isolierung von 4,4'-Dihydroxydiphenylsulfon aus dem Reaktionsansatz mitverwenden, wie beispielsweise in JP-A 50/106 937, CN-A 87/100 796, EP-A 220 004 oder WO 92/02493 beschrieben, sind ebenfalls aus ökologischen und sicherheitstechnischen Gründen bedenklich.

Aus der US-A 3 297 766 ist ein semikontinuierliches Verfahren zur Herstellung von 4,4'-Dihydroxydiphenylsulfon bekannt, bei dem Schwefelsäure mit Phenol bei 180-188°C unter fortwährendem Abdestillieren des Reaktionswassers umgesetzt, die Reaktionsmischung danach auf 55-75°C abgekühlt und der ausgefallene Feststoff abfiltriert und mit Wasser gewaschen wird. Man erhält so eine Mischung aus 4,4'- und 2,4'-Dihydroxydiphenylsulfon. Die Abfallströme aus der Abfiltration und dem Nachwaschen sowie das beim Abdestillieren des Reaktionswassers ausgetragenen Phenol werden in den nächsten Zyklus des Herstellprozesses zurückgeführt.

In der JP-A 50/106 936 wird die Herstellung von 4,4'-Dihydroxydiphenylsulfon durch Umsetzung von Phenol mit einem Sulfonierungsmittel wie Schwefelsäure bei 180-200°C und Auskristallisation und Abtrennung des 4,4'-Dihydroxydiphenylsulfons beschrieben. Zur Isolierung des 4,4'-Dihydroxydiphenylsulfons wird das rohe Umsetzungsprodukt in einer wäßrigen 3 bis 8 gew.-%igen Phenol-Lösung gelöst oder dispergiert und das 4,4'-Dihydroxydiphenylsulfon mit oder ohne Kühlung auskristallisiert und abgetrennt. Wenn im Reaktionsprodukt nicht umgesetztes Phenol noch in größerer Menge vorhanden ist, kann auch nur mit dem Zusatz von reinem Wasser gearbeitet werden. Pro Gewichtsteil Reaktionsprodukt werden 1 bis 5 Gewichtsteile wäßrige Phenol-Lösung verwendet. Die abgetrennte Lösung, welche Phenol und Wasser enthält, kann in einem nachfolgenden Reaktionszyklus wiederverwendet werden. Gemäß dem Ausführungsbeispiel der JP-A 50/106 936 erzielt man bei Behandlung des Reaktionsproduktes mit 75°C heißer, wäßriger, 8 gew.-%iger Phenol-Lösung, Abkühlung auf 40°C und Abzentrifugieren des ausgefallenen 4,4'-Dihydroxydiphenylsulfons eine Isomerenreinheit von 98,7 %.

Das Verfahren der JP-A 50/106 936 liefert zwar das gewünschte Produkt in hoher Isomerenreinheit, hat jedoch den Nachteil, daß es mit großen Mengen an wäßriger Behandlungslösung für die Isolierung aus dem Reaktionsansatz arbeitet. Diese Mengen erfordern zum einen großdimensionierte und daher teure Apparaturen, zum anderen wird dadurch die Ausbeute gemindert. Weiterhin hat die Anwesenheit großer Mengen an Phenol bei der Isolierung des Produktes den Nachteil, daß das Phenol leicht unter Bildung von Addukt-Komplexen mit dem Produkt als Verunreinigung in diesem haftenbleiben kann.

Es ist bekannt, daß sich bei Sulfonierungsreaktionen von Phenol mit Schwefelsäure reversible Gleichgewichte einstellen, die über die 2- und die 4-Phenolsulfonsäure mit weiterem Phenol zu 2,4'-und 4,4'-Dihydroxydiphenylsulfon führen. Das zwischen 2,4'- und 4,4'-Dihydroxydiphenylsulfon bestehende Isomerisierungsgleichgewicht liegt auf der Seite des thermodynamisch stabileren 4,4'-Isomeren. Die Isomerisierung ist jedoch kinetisch gehemmt und es bedarf meist eines Katalysators wie beispielsweise 4-Phenolsulfonsäure, damit sich das Gleichgewicht innerhalb eines verfahrenstechnisch akzeptablen Zeitraumes einstellt. Das Isomerisierungsgleichgewicht kann weiterhin durch selektiven Entzug des 4,4'-Isomers auf dessen Seite verschoben werden. Weiterhin können sich bei einem Unterschuß an Phenol auch Trimere wie 2,4',4"-Trihydroxytriphenyldisulfon sowie höhere Oligomere der Phenolsulfonsäure bilden.

Aufgabe der vorliegenden Erfindung war es, ein bei den Stufen der Umsetzung und der Isolierung des Produktes aus dem Reaktionsansatz von inerten organischen Lösungsmitteln freies Herstellverfahren für 4,4'-Dihydroxydiphenylsulfon bereitzustellen, bei dem die Abfallströme in den Herstellprozeß zurückgeführt werden können, das das gewünschte Produkt in hoher Isomerenreinheit und hoher Ausbeute auch ohne nachgeschaltete Reinigungsoperationen liefert und das bei der Isolierung des Produktes aus dem Reaktionsansatz mit möglichst geringen Mengen an Behandlungslösung, die weitgehend frei von nicht umgesetztem Phenol sein sollte, auskommt.

Demgemäß wurde das eingangs definierte semikontinuierliche Verfahren zur Herstellung von 4,4'-Dihydroxydiphenylsulfon gefunden, welches dadurch gekennzeichnet ist, daß man bei der Durchführung des Schrittes (b) Rohprodukt und Wasser im Gew.-Verhältnis von 85:15 bis 55:45, vorzugsweise 75:25 bis 60:40, einsetzt.

Das zum Suspendieren des Reaktionsansatzes verwendete heiße Wasser ist frei von inerten organischen Lösungsmitteln, insbesondere wassermischbaren wie Methanol, Ethanol, Isopropanol oder Aceton oder gegebenenfalls halogenierten Kohlenwasserstoffen wie Toluol, Chlorbenzol oder Dichlorbenzolen. Die Restmengen an nicht umgesetztem Phenol, die aus dem Reaktionsansatz beim Suspendieren herausgelöst werden können oder zu einem geringen Teil auch im eingesetzten Wasser schon vorhanden sein können, wenn dieses aus dem vorangegangenen Zyklus des Herstellprozesses stammt und durch Abdestillieren der Mutterlauge aus dem dortigen Schritt (b) erzeugt wurde, können bis maximal 2,5 Gew.-%, insbesondere 0,05 bis 1,5 Gew.-%, vor allem 0,1 bis 1,0 Gew.-%, jeweils bezogen auf die eingesetzte Wassermenge, betragen.

In einer bevorzugten Ausführungsform weist das zur Behandlung des Reaktionsansatzes in Schritt (b) zugegebene Wasser eine Temperatur von 80°C bis 100°C auf. Besonders gute Ergebnisse erzielt man mit Wasser von 90°C bis 100°C. Im Prinzip kann man auch bei Temperaturen über 100°C, beispielsweise bei 100°C bis 130°C, arbeiten, wenn man die Behandlung mit Wasser in einer geschlossenen Apparatur unter Druck durchführt. Die Zugabe des Behandlungswassers kann auch in Form von Wasserdampf erfolgen. Die Dauer der Behandlung mit Wasser ist unkritisch, in der Regel liegt diese bei 5 Minuten bis 5 Stunden. Normalerweise erfolgt während der Behandlung mit Wasser eine mechanische Durchmischung, beispielsweise durch Rühren.

In einer weiteren bevorzugten Ausführungsform führt man in Schritt (b) das Suspendieren des Rohproduktes in Wasser und das Abfiltrieren des Produktes bei annähernd der gleichen Temperatur durch. Annähernd gleiche Temperatur bedeutet üblicherweise das Arbeiten in einem Temperaturinterval von ca. 10°C, insbesondere ca. 5°C. Bei dieser Ausführungsform wird eine Abkühlung der Suspension vermieden, die ein Auskristallisieren von gelösten Anteilen zur Folge hätte.

Das Suspendieren ("Aufschlämmen") und das Abfiltrieren können mit hierzu üblichen Techniken und Apparaten wie beispielsweise Rührkesseln, Mischern oder Knetern für das Suspendieren und Filternutschen oder Zentrifugen für das Abfiltrieren durchgeführt werden.

In einer weiteren bevorzugten Ausführungsform wäscht man das in Schritt (b) abfiltrierte Produkt mit mindestens 40°C, vorzugsweise 80°C bis 100°C, insbesondere 90°C bis 100°C heißem Wasser nach. Die Menge des zum Nachwaschen verwendeten Wasser sollte möglichst gering sein und liegt in der Regel bei maximal 100 %, vorzugsweise maximal 85 % des zum Suspendieren des Reaktionsansatzes eingesetzten Wassers.

Ganz besonders bevorzugt wird eine Durchführung des Schrittes (b), bei der man das Suspendieren des Rohproduktes in Wasser und das Abfiltrieren des Produktes bei annähernd der gleichen Temperatur in einem Temperaturbereich von 80°C bis 100°C, insbesondere 90°C bis 100°C, durchführt und mit maximal 100 %, insbesondere maximal 85 % des zum Suspendieren des Reaktionsansatzes eingesetzten Wassers, welches auch annähernd die gleiche Temperatur wie beim Suspendieren und Abfiltrieren aufweist, nachwäscht.

Ein Vorteil des erfindungsgemäßen Verfahren besteht darin, daß das gemäß den vorgenannten Maßnahmen in Schritt (b) hergestellte Produkt in der Regel bereits eine durchschnittliche Reinheit von >96 %, insbesondere >97,5 %, und eine durchschnittliche Isomerenreinheit von >98 %, insbesondere von >98,4 %, besitzt. Diese hohe Reinheit und Isomerenreinheit werden dadurch erreicht, daß im Gegensatz zu allen bisher bekannten Verfahren die während der Umsetzung in Schritt (a) gebildeten, bereits sehr reinen Rohkristalle des 4,4'-Dihydroxydiphenylsulfons nicht zerstört werden, sondern durch das heiße Behandlungswasser von dem in der Mutterlauge besser löslichen und darin angereicherten 2,4'-Dihydroxydiphenylsulfon befreit werden. Die höhe Selektivität der Prozeßführung bedingt aufgrund der Löslichkeit von 4,4'-Dihydroxydiphenylsulfon zwar einen Ausbeuteverlust, welcher durch die Rückführung der Abfallströme gemäß Schritt (c) und die semikontinuierliche Prozeßführung letztendlich jedoch wieder ausgeglichen wird.

Ein weiterer vorteil des erfindungsgemäßen Verfahrens besteht darin, daß die hauptsächlich das 2,4'-Dihydroxydiphenylsulfon enthaltende Mutterlauge aus Schritt (b) nach Abdestillieren des Wassers zu Beginn des nächsten Zyklusses des Herstellprozesses in Schritt (a) unter geschickter Ausnutzung des chemischen Gleichgewichtes wieder eingesetzt wird. Dadurch wird der Rückstand der Mutterlauge in Phenolsulfonsäure umgewandelt. Das abdestillierte Wasser kann ebenfalls im nächsten Zylkus wieder in Schritt (b) eingesetzt werden.

Das erfindungsgemäße Verfahren zeichnet sich außerdem durch eine hohe Gesamtausbeute vom meist über 90 %, insbesondere von 92 % oder mehr, jeweils bezogen auf eingesetztes Phenol, aus.

Das nach dem erfindungsgemäßen Verfahren hergestellte 4,4'-Dihydroxydiphenylsulfon zeichnet sich auch durch seine gutes Kristallisationsverhalten aus, d.h. die erhaltenen Kristalle sind groß und gut ausgebildet sowie staubarm, so daß sie gut handhabbar und dosierbar sind.

Als Sulfonierungsmittel in Schritt (a) setzt man vorzugsweise konzentrierte Schwefelsäure, Oleum ("rauchende" Schwefelsäure) oder Schwefeltrioxid ("Schwefelsäureanhydrid") ein. Besonders bevorzugt wird konzentrierte Schwefelsäure mit einem Gehalt von ca. 70 bis 100 Gew.-%, beispielsweise die handelsübliche ca. 96 gew.-%ige Schwefelsäure.

Der Schritt (a) des erfindungsgemäßen Verfahrens kann wie nachfolgend beschrieben durchgeführt werden.

Phenol und das Sulfonierungsmittel können gemeinsam vorgelegt und dann auf Umsetzungstemperatur gebracht werden. Das Phenol kann jedoch auch portionsweise oder kontinuierlich im Verlauf der Umsetzung, insbesondere nach der Aufheizphase, zudosiert werden.

Die Umsetzungstemperatur liegt üblicherweise bei 140°C bis 230°C, insbesondere bei 150°C bis 200°C, vor allem bei 160°C bis 175°C. Bei einer Umsetzungstemperatur von weniger als 165°C wird zweckmäßigerweise bei vermindertem Druck gearbeitet, um das Sieden des Phenols zu gewährleisten.

Das molare Verhältnis von Phenol zum Sulfonierungsmittel, beispielsweise Schwefelsäure, beträgt bei der gesamten Umsetzung in Schritt (a) normalerweise 1,6 : 1 bis 25 : 1, vorzugsweise 1,8 : 1 bis 10 : 1, insbesondere 1,9 : 1 bis 2,5 : 1.

Das bei der Sulfonierung von Phenol mit Schwefelsäure bzw. Phenolsulfonsäure entstehende Wasser kann mittels von in der Reaktionsmischung vorhandenem, überschüssigem Phenol laufend azeotrop aus der Umsetzung abdestilliert werden und das Phenol nach Phasenabscheidung des Wasser wieder in den Reaktor zurückgeführt werden. Nach der azeotropen Abdestillation ("Auskreisung") von in der Regel mindestens 50 %, jedoch besser 90 bis 100 % der sich nach der Theorie bildenden Wassermenge wird im allgemeinen kein Phenol mehr in die Umsetzung zurückgeführt, sondern das Phenol nach und nach bei zweckmäßigerweise 160°C bis 200°C abdestilliert. Das nicht umgesetzte Phenol wird im nächsten Zyklus des Herstellprozesses wieder eingesetzt.

In einer bevorzugten Ausführungsform wird in Schritt (a) zuerst Phenol mit dem Sulfonierungsmittel weitgehend zu Phenolsulfonsäure umgesetzt, das hierbei gegebenenfalls entstehende Reaktionswasser durch Destillation entfernt, danach die Phenolsulfonsäure mit weiterem Phenol zum Dihydroxydiphenylsulfon umgesetzt und hierbei entstehendes Reaktionswasser wiederum durch Destillation entfernt.

Danach schließt sich in der Regel eine Isomerisierungphase des Reaktionsansatzes bei einer Temperatur von üblicherweise 140°C bis 180°C, insbesondere 150°C bis 170°C, und einer Isomerisierungszeit von normalerweise 0,1 bis 10 Stunden, insbesondere 1 bis 4 Stunden, an. Während dieser Isomerisierungsphase verschiebt sich das Gleichgewicht auf die Seite des 4,4'-Dihydroxydiphenylsulfons und die Menge an 2,4'-Dihydroxydiphenylsulfon nimmt ab. Die Isomerisierung wird in der Regel durch geringe Mengen an Phenolsulfonsäure, welche üblicherweise noch in geringen Mengen im Reaktionsansatz vorhanden ist, insbesondere wenn mit einem leichten stöchiometrischen Überschuß an Sulfonierungsmittel gearbeitet wird, katalysiert.

In einer bevorzugten Ausführungsform wird nach Durchführung der Umsetzung von Schritt (a), jedoch in der Regel vor Durchführung einer Isomerisierungsphase, überschüssiges Phenol praktisch vollständig durch Destillation entfernt.

Vor Zugabe des heißen Behandlungswassers gemäß Schritt (b) wird der Reaktionsansatz üblicherweise auf Temperaturen unter 130°C abgekühlt.

Das Rückführen der entstehenden edukt- und/oder produkthaltigen Abfallströme in den Herstellprozeß, d.h. nach Abdestillation von Wasser in den nächsten Zyklus des Herstellprozesses, gemäß Schritt (c) erfolgt vorzugsweise geschlossen, d.h. vollständig bezogen auf eingesetztes Phenol, die Phenolsulfonsäure als Zwischenprodukt und die Dihydroxydiphenylsulfone. Die Rückführung von mit Wasser in Schritt (a) und aus der Mutterlauge in Schritt (b) azeotrop abdestilliertem Phenol, welches im Kondensat gelöst bleibt und sich nicht als separate Phase durch übliche Phasentrennung abtrennen läßt, gestaltet sich meist unwirtschaftlich - zumal da diese Phenolmengen vernachlässigbar gering sind - und kann damit entfallen.

Unter semikontinuierlicher Verfahrensführung ist die mehrfache Wiederholung ("Zyklen-Fahrweise") von nicht kontinuierlichen Verfahrensschritten mit Rückführung von Prozeßströmen zu verstehen. Die Anzahl der Zyklen, die das erfindungsgemäße semikontinuierliche Verfahren durchlaufen kann, ist im Prinzip nicht begrenzt. Üblicherweise hat sich nach dem dritten oder vierten Zyklus eine quasi-stationäres Gleichgewicht bezüglich der Kenndaten der zurückgeführten Abfallströme und der Reinheits- und Umsatzdaten der Gesamtumsetzung eingestellt. Bemerkenswerterweise reichern sich - soweit dies nach Durchlaufen von ca. 10 Zyklen erkennbar ist - keine Verunreinigungen oder Nebenprodukte an.

Das in Schritt (b) anfallende 4,4'-Dihydroxydiphenylsulfon kann durch übliches Umkristallisieren noch weiter gereinigt werden. Vorzugsweise erfolgt diese Umkristallisation aus einer Wasser-Aceton-Mischung, die in der Regel ein Volumen-Verhältnis von 50:50 bis 97:3, insbesondere 80:20 bis 95:5, aufweist. Anstelle von Aceton können jedoch auch andere wasserlösliche oder wassermischbare organische Lösungsmittel, z.B. aliphatische Alkohole mit 1 bis 3 C-Atomen wie Methanol, Ethanol, n-Propanol oder Isopropanol, Dimethylsulfoxid, Dimethylformamid, Dioxan, Ethylenglykol, Propylenglykol oder höhere Ethylen- oder Propylenglykole mit bis zu 3 Glykoleinheiten, in üblichen Mischungsverhältnissen mit Wasser verwendet werden. Vorteilhaft ist oft auch die Mitverwendung von Aktivkohle bei der Umkristallisation. Das so erhaltene Reinprodukt weist in der Regel eine Isomerenreinheit an 4,4'-Dihydroxydiphenylsulfon von mindestens 99,7 % auf.

Das nachfolgende Beispiel soll die vorliegende Erfindung verdeutlichen, ohne sie zu beschränken.

### Beispiel

### Schritt (a)

512 g Mutterlauge aus dem Schritt (b) des vorangegangenen Zyklus (nicht flüchtiger Anteil: 32,2 Gew.-%) wurden innerhalb von 2,5 Stunden bei 110°C im Vakuum durch Abdestillieren des Wassers aufkonzentriert. Hierzu wurden 346 g (3,7 mol) Phenol und anschließend 437 g (4,3 mol) 96 gew.-%ige Schwefelsäure zudosiert. Danach wurde 1 Stunde bei 160°C gerührt. Dann wurde ein Vakuum von 150 mbar eingestellt und das entstandene Reaktionswasser innerhalb von 3 Stunden ausgekreist.

45,4 g Restphenol aus dem vorangegangenen Zyklus (nicht flüchtiger Anteil: 72,5 Gew.-%, 0,4 mol) und 472 g (5,0 mol) frisches Phenol wurden bei 165°C und Normaldruck zum Reaktionsansatz dosiert. Das Vakuum wurde auf 300 mbar erniedrigt und das Reaktionswasser innerhalb von 6 Stunden ausgekreist. Danach wurde das überschüssige Phenol so vollständig wie möglich abdestilliert. Insgesamt bildeten sich 180 g phenolhaltiges Reaktionswasser (nicht flüchtiger Anteil: 9,8 Gew.-%, 0,2 mol Phenol) und 62 g wäßriges Restphenol (nicht flüchtiger Anteil: 74 Gew.-%, 0,5 mol Phenol).

Der Reaktionsansatz wurde mit Stickstoff belüftet und die Temperatur schrittweise innerhalb einer Stunde von 165°C auf 140°C gesenkt. Anschließend wurde noch 3 Stunden bei dieser Temperatur isomerisiert.

### Schritt (b)

Zum 100°C heißen Reaktionsansatz (1223 g) wurden 583 g 90°C heißes Wasser, welches durch Abdestillieren aus der Mutterlauge aus Schritt (b) des vorangegangenen Zyklus erhalten wurde, gegeben und der Reaktionsansatz wurde durch 15minütiges Rühren bei 90-95°C aufgeschlämmt. Anschließend wurde die Suspension bei 90°C filtriert und dreimal mit jeweils 160 g 90°C heißem Wasser nachgewaschen. Es wurden 1120g Kristallisat (4,5 mol Dihydroxydiphenylsulfon, bezogen auf frisch eingesetztes Phenol) erhalten.

### Umkristallisation

Das erhaltene Kristallisat wurde in 7200 ml einer Mischung aus Wasser und Aceton im Vol.-Verhältnis von 90:10 bei 80°C unter Mitverwendung von Aktivkohle umkristallisiert. Dazu wurde das Kristallisat bei 80°C gelöst, mit 3,2 g handelsüblicher Aktivkohle versetzt und bei dieser Temperatur gerührt. Nach Klarfiltration durch ein beheiztes Druckfilter entstand eine farblose Lösung. Nach Abkühlung auf 25°C wurden die farblosen Kristalle abgesaugt und zweimal mit jeweils 200 g Wasser gewaschen. Man erhielt 1031 g (4,1 mol, 95 % bezogen auf frisch eingesetztes Phenol) farbloses Reinkristallisat.

Nach Durchführung von 10 Zyklen analog zu der oben beschriebenen Prozeßführung wurden eine Gesamtausbeute an 4,4'-Dihydroxydiphenylsulfon von 92 %, bezogen auf eingesetztes Phenol, und eine durchschnittliche Reinheit von 98,4 % (Produkt aus Schritt (b)) bzw. 99,7 % nach Umkristallisation erzielt.

## Patentansprüche

1. Semikontinuierliches Verfahren zur Herstellung von 4,4'-Dihydroxydiphenylsulfon mit den Schritten
(a) Umsetzen von Phenol mit einem Sulfonierungsmittel,
(b) Suspendieren des entstandenen Rohproduktes in mindestens 40°C heißem Wasser, welches frei von inerten organischen Lösungsmitteln ist und Restmengen an nicht umgesetztem Phenol enthalten kann, und Abfiltrieren des Produktes sowie
(c) Rückführen der entstehenden edukt- und/oder produkthaltigen Abfallströme in den Herstellprozeß,
**dadurch gekennzeichnet, daß** man bei Durchführung des Schrittes (b) Rohprodukt und Wasser im Gew.-Verhältnis von 85:15 bis 55:45 einsetzt.

2. Verfahren nach Anspruch 1, **dadurch gekennzeichnet, daß** die Restmengen an nicht umgesetztem Phenol im Wasser in Schritt (b) maximal 2,5 Gew.-% betragen.

3. Verfahren nach Anspruch 1 oder 2, **dadurch gekennzeichnet, daß** das Wasser in Schritt (b) eine Temperatur von 80°C bis 100°C aufweist.

4. Verfahren nach den Ansprüchen 1 bis 3, **dadurch gekennzeichnet, daß** man in Schritt (b) das Suspendieren des Rohproduktes in Wasser und das Abfiltrieren des Produktes bei annähernd der gleichen Temperatur durchführt.

5. Verfahren nach den Ansprüchen 1 bis 4, **dadurch gekennzeichnet, daß** man in Schritt (b) das abfiltrierte Produkt mit mindestens 40°C heißem Wasser nachwäscht.

6. Verfahren nach den Ansprüchen 1 bis 5, **dadurch gekennzeichnet, daß** man in Schritt (a) als Sulfonierungsmittel konzentrierte Schwefelsäure, Oleum oder Schwefeltrioxid einsetzt.

7. Verfahren nach den Ansprüchen 1 bis 6, **dadurch gekennzeichnet, daß** man in Schritt (a) zuerst Phenol mit dem Sulfonierungsmittel weitgehend zu Phenolsulfonsäure umsetzt, das hierbei gegebenenfalls entstehende Reaktionswasser durch Destillation entfernt, danach die Phenolsulfonsäure mit weiterem Phenol zum Dihydroxydiphenylsulfon umsetzt und hierbei entstehendes Reaktionswasser wiederum durch Destillation entfernt.

8. Verfahren nach den Ansprüchen 1 bis 7, **dadurch gekennzeichnet, daß** man nach Durchführung der Umsetzung von Schritt (a) überschüssiges Phenol praktisch vollständig durch Destillation entfernt.

9. Verfahren nach den Ansprüchen 1 bis 8, **dadurch gekennzeichnet, daß** man das in Schritt (b) erhaltene 4,4'-Dihydroxydiphenylsulfon durch Umkristallisation aus einer Wasser-Aceton-Mischung weiter reinigt.

## Claims

1. A semicontinuous process for the preparation of 4,4'-dihydroxydiphenyl sulfone, comprising the following steps:
(a) reaction of phenol with a sulfonating agent,
(b) suspension of the resulting crude product in water heated to at least 40°C, which is free of inert organic solvents and may comprise residual amounts of unreacted phenol, and filtration of the product, and
(c) recycling of the resulting educt-containing and/or product-containing waste streams into the preparative process,
wherein, in carrying out step (b), the crude product and water are used in a weight ratio of 85:15 to 55:45.

2. The process according to claim 1 wherein, in step (b), the residual amounts of unreacted phenol in the water are at most 2.5% by weight.

3. The process according to claim 1 or 2 wherein the water in step (b) is at a temperature of 80°C to 100°C.

4. The process according to any of claims 1 to 3 wherein, in step (b), the suspension of the crude product in water and the filtration of the product are carried out at approximately the same temperature.

5. The process according to any of claims 1 to 4 wherein, in step (b), the product filtered off is rinsed with water heated to at least 40°C.

6. The process according to any of claims 1 to 5 wherein the sulfonating agent used in step (a) is concentrated sulfuric acid, oleum or sulfur trioxide.

7. The process according to any of claims 1 to 6 wherein, in step (a), firstly phenol is extensively reacted with the sulfonating agent to give phenolsulfonic acid, any water of reaction thereby formed is removed by distillation, the phenolsulfonic acid is then reacted with more phenol to give dihydroxydiphenyl sulfone and the water of reaction thereby formed is again removed by distillation.

8. The process according to any of claims 1 to 7 wherein (a) excess phenol is virtually completely removed by distillation after the reaction of step.

9. The process according to any of claims 1 to 8 wherein the 4,4'-dihydroxydiphenyl sulfone obtained in step (b) is further purified by recrystallization from a water/acetone mixture.

## Revendications

1. Procédé semi-continu de fabrication de 4,4'-dihydroxydiphénylsulfone avec les étapes suivantes :
(a) réaction de phénol avec un agent de sulfonation,
(b) suspension du produit brut obtenu dans de l'eau chaude au moins à 40°C, qui est exempte de solvants organiques inertes et peut contenir des quantités résiduelles de phénol qui n'a pas réagi, et séparation par filtration du produit, ainsi que
(c) recyclage des flux résiduels contenant des éduits et/ou des produits dans le processus de fabrication,
**caractérisé en ce que** l'on utilise, lors de la réalisation de l'étape (b), du produit brut et de l'eau dans le rapport pondéral de 85:15 à 55:45.

2. Procédé selon la revendication 1, **caractérisé en ce que** les quantités résiduelles de phénol qui n'a pas réagi dans de l'eau à l'étape (b) atteignent au maximum 2,5% en poids.

3. Procédé selon la revendication 1 ou 2, **caractérisé en ce que** l'eau à l'étape (b) présente une température de 80°C à 100°C.

4. Procédé selon les revendications 1 à 3, **caractérisé en ce que** l'on réalise à l'étape (b) la suspension du produit brut dans de l'eau et la séparation par filtration du produit à peu près à la même température.

5. Procédé selon les revendications 1 à 4, **caractérisé en ce qu'**on lave ultérieurement à l'étape (b) le produit séparé par filtration avec de l'eau chaude au moins à 40°C.

6. Procédé selon les revendications 1 à 5, **caractérisé en ce que** l'on utilise à l'étape (a) comme agent de sulfonation de l'acide sulfurique concentré, de l'oléum ou du trioxyde de soufre.

7. Procédé selon les revendications 1 à 6, **caractérisé en ce que** l'on convertit à l'étape (a) tout d'abord du phénol avec l'agent de sulfonation largement en acide phénolsulfonique, on élimine l'eau réactionnelle éventuellement produite en l'occurrence par distillation, ensuite l'acide phénolsulfonique est converti avec une quantité supplémentaire de phénol en dihydroxydiphénylsulfone et on élimine l'eau réactionnelle produite en l'occurrence à nouveau par distillation.

8. Procédé selon les revendications 1 à 7, **caractérisé en ce que**, après avoir réalisé la conversion de l'étape (a), on élimine le phénol en excès pratiquement complètement par distillation.

9. Procédé selon les revendications 1 à 8, **caractérisé en ce que** l'on épure encore le 4,4'-dihydroxydiphénylsulfone obtenu à l'étape (b) par recristallisation à partir d'un mélange d'eau et d'acétone.
